(19) **Europäisches Patentamt**
**European Patent Office**
**Office européen des brevets**

(11) **EP 3 932 409 A1**

(12) # EUROPEAN PATENT APPLICATION

(43) Date of publication:
**05.01.2022 Bulletin 2022/01**

(51) Int Cl.:
*A61K 31/546* *(2006.01)*  *A61K 31/7048* *(2006.01)*
*A61P 11/00* *(2006.01)*  *A61P 31/14* *(2006.01)*

(21) Application number: **20382570.8**

(22) Date of filing: **29.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Consejo Superior de Investigaciones Científicas (CSIC)**
  **28006 Madrid (ES)**
- **Universitat de València**
  **46010 Valencia (ES)**

- **Universitat Politècnica de València**
  **46022 Valencia (ES)**

(72) Inventors:
- **BENLLOCH BAVIERA, Jose María**
  **46022 Valencia (ES)**
- **GÁLVEZ LLOMPART, María**
  **46022 Valencia (ES)**
- **GÁLVEZ ALVAREZ, Jorge**
  **46010 Valencia (ES)**
- **ZANNI, Riccardo**
  **46010 Valencia (ES)**

(74) Representative: **Pons**
**Glorieta Rubén Darío 4**
**28010 Madrid (ES)**

(54) **COMPOUNDS FOR THE TREATMENT AND PREVENTION OF VIRAL INFECTIONS CAUSED BY CORONAVIRUSES**

(57)    The invention relates to a compound or to a pharmaceutical composition thereof for their use in the treatment and/or prevention of viral infections caused by coronaviruses, particularly to COVID-19 disease caused by SARS-CoV-2 coronavirus. The invention also relates to an antiviral which comprises said compounds or pharmaceutical compositions thereof.

EP 3 932 409 A1

**Description**

The invention relates to a compound or to a pharmaceutical composition thereof for their use in the treatment and/or prevention of viral infections caused by coronaviruses, particularly to COVID-19 disease caused by SARS-CoV-2 coronavirus. The invention also relates to an antiviral which comprises said compounds or pharmaceutical compositions thereof.

**BACKGROUND ART**

In the face of the SARS-CoV-2 pandemic spread, a rapid search for precise treatments of COVID-19 and associate or related diseases becomes essential.

Some proposals for drug repurposing for the treatment of coronaviruses have been published using the usual techniques of drug design, mainly molecular docking, complemented by other stories such as molecular mechanics (see Wang J. Fast Identification of Possible Drug Treatment of Coronavirus Disease -19 (COVID-19) Through Computational Drug Repurposing Study. J Chem Inf Model https://doi.org/10.1021/acs.jcim.0c00179 (2020)).

Gautret et al (see Gautret P, Lagier JC, Parola P, Hoang VT, Meddeb L, et al. Hydroxychloroquine and azithromycin as a treatment of COVID-19: results of an open-label non-randomized clinical trial. Int J Antimicrob Agents (https://doi.org/10.1016/j.ijantimicag.2020.105949) has also described the use of azithromycin in patients affected by COVID-19 disease as a preventive of bacterial complications combined with hydroxychloroquine, although is silent about its actual activity as antiviral.

On the other hand, molecular topology (MT) has proven to be a very powerful tool for the design and selection of new lead drugs, in fields as diverse as cancer, malaria (see Zanni R, Galvez-Llompart M, Garcia-Domenech R, Galvez J. Latest advances in molecular topology applications for drug discovery. Expert Opin Drug Discov 10 (9), 945-957 (2015)) or Alzheimer's (Wang J, Land D, Ono K, Galvez J, Zhao W, et al. Molecular topology as novel strategy for discovery of drugs with aβ lowering and anti-aggregation dual activities for Alzheimer's disease. PloS one 9 (3). (2014)) to mention just a few.

Basically, the MT method is based on characterizing the molecules by a set of numbers, called topological indices, which depend on the structure of each molecule and hence on its pharmacological activity. This way, it is possible to gather the molecules in different mathematical patterns depending on their activity. For example, we may get the anticancer pattern and even the anticancer pattern associated with a given mechanism of action (for example, topoisomerase inhibition).

Once a mathematical pattern has been achieved it is not difficult to search for new molecules matching the same pattern and therefore showing the same pharmacological activity, but with improved pharmacodynamic, pharmacokinetic or toxicological profiles (more potency, better absorption, lower toxicity, etc.). By using such a pattern, it is possible to identify a great deal of molecules potentially active in that particular field.

Accordingly, it is desirable to provide a fast, effective and safe treatment and/or prevention for viral infections caused by coronaviruses, particularly for the COVID-19 disease caused by SARS-CoV-2 virus by repositioning of drugs using a computational study based on molecular topology (TM).

**SUMMARY OF THE INVENTION**

An aspect of the present invention relates to a compound selected from clarithromycin, lexithromycin, cefuroxime, nonactine, cefbuperazone, azithromycin, erythromycin, diproleandomycin. flurithromycin, neutramycin, mirosamicin, fomidacillin, cefclidin, cethromycin, dalbavancin, nogalamycin, paldimycin, paulomycin, mannosidostreptomycin, bluensomycin, lividomycin, mideplanin (Mdl-62,873), teicoplanin, roxithromycin, spiramycin, draxxin, berytromycin, tulathromycin, oleandomycin, kitasamycin, roquitamycin, maridomycin or a pharmaceutically acceptable salt thereof for use in the treatment and/or prevention of a viral infection, preferably for the treatment and/or prevention of a viral infection caused by coronavirus, more preferably for the treatment and/or prevention of viral infections caused by SARS-CoV-2, and even more preferably for the treatment and/or prevention of COVID-19 disease.

In another embodiment the invention relates to the compound or a pharmaceutically acceptable salt thereof for the use as defined above, wherein the compound is selected from clarithromycin, lexithromycin, cefuroxime, nonactine, cefbuperazone, azithromycin, erythromycin, diproleandomycin. flurithromycin, neutramycin, mirosamicin, fomidacillin, cefclidin and cethromycin.

In another embodiment the invention relates to the compound or a pharmaceutically acceptable salt thereof for the use as defined above, wherein the compound is selected from clarithromycin, lexithromycin, cefuroxime and erythromycin.

In another embodiment the invention relates to the compound or a pharmaceutically acceptable salt thereof for the use as defined above, wherein the compound is selected from lexithromycin, clarithromycin and cefuroxime.

In another embodiment the invention relates to the compound or a pharmaceutically acceptable salt thereof for the use as defined above, wherein the compound is selected from lexithromycin and clarithromycin.

Throughout the present specification, by the term "treatment" is meant eliminating, reducing or ameliorating the cause or the effects of a disease. For purposes of this invention treatment includes, but is not limited to, alleviation, amelioration or elimination of one or more symptoms of the disease; diminishment of the extent of the disease; stabilized (i.e. not worsening) state of disease; delay or slowing of disease progression; amelioration or palliation of the disease state; and remission of the disease (whether partial or total).

As used herein, "prevention" refers to preventing the occurrence of a disease in a subject that is predisposed to or has risk factors but does not yet display symptoms of the disease. Prevention includes also preventing the recurrence of a disease in a subject that has previously suffered said disease.

The term "viral infection" refers to the proliferation of a harmful virus inside the body. Viruses cannot reproduce without the assistance of a host. Viruses infect a host by introducing their genetic material into the cells and hijacking the cell's internal machinery to make more virus particles.

"Coronaviruses" refers to a group (Family) of viruses that has a single, positive sense, linear stranded RNA ssRNA molecule of 25 to 33 kb. The term coronavirus includes any member of the Coronaviridae family, preferably Orthocoronaviridae, and more preferably of the genus Betacoronavirus and even more preferably, SARS-CoV or SARS-CoV-2.

"SARS-CoV-2" refers to Severe acute respiratory syndrome coronavirus 2, and it is the strain of coronavirus that causes coronavirus disease 2019 (COVID-19), the respiratory illness responsible for the COVID-19 pandemic.

The "covid-19 disease" refers to the disease caused by the SARS-CoV-2 infection. Common symptoms include fever, cough, fatigue, shortness of breath, and loss of sense of smell. Complications may include pneumonia and acute respiratory distress syndrome. The recent emergence of this disease gives us a shallow knowledge of the different symptoms and the biology of the infection, some recent reviews point to a three-stage disease: asymptomatic, pulmonary and hyperinflammation (McGonagle, D., O'Donell, J., et al. Immune mechanisms of pulmonary intravascular coagulopathy in COVID-19 pneumonia. Lancet Rheumatol 2020 https://doi.org/10.1016/S2665-9913(20)30121-1; Tay, M.Z., Poh, C.M., Rénia, L. et al. The trinity of COVID-19: immunity, inflammation and intervention. Nat Rev Immunol 20, 363-374 (2020)) points to different phases for the disease).

The term pharmaceutically acceptable salt represents those salts which are, according to medical judgment, suitable for use in contact with the tissues of humans and other mammals without undue toxicity, irritation, allergic response and the like. Pharmaceutically acceptable salts are well known in the art.

The present invention also relates to a pharmaceutical composition for use in the treatment and/or prevention of viral infections and/or associated diseases, preferably for the treatment and/or prevention of viral infections associated to coronavirus and/or associated diseases, more preferably for the treatment and/or prevention of viral infections associated to SARS-CoV-2 and/or associated diseases, even more preferably for the treatment and/or prevention of a disease associated to a viral infection and/or associated diseases, that comprises a compound as defined above (or a pharmaceutically acceptable salt or solvate thereof) and one or more pharmaceutically acceptable excipients. The excipients must be "acceptable" in the sense of being compatible with the other ingredients of the composition and not deleterious to the recipients thereof.

Another aspect of the present invention relates to an antiviral which comprises the compound as defined above or a pharmaceutical composition thereof.

Another aspect of the present invention relates to the antiviral as defined above for use in the treatment and/or prevention of for use in the treatment and/or prevention of a viral infection, preferably for the treatment and/or prevention of a viral infection caused by coronavirus, more preferably for the treatment and/or prevention of viral infections caused by SARS-CoV-2, and even more preferably for the treatment and/or prevention of COVID-19 disease.

The compounds of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

The dosage and frequency of doses will depend upon the nature and severity of the disease to be treated, the age, the general condition and body weight of the patient, as well as the particular compound administered and the route of administration, among other factors. A representative example of a suitable dosage range is from about 0.01 mg/Kg to about 100 mg/Kg per day, which can be administered as a single or divided doses.

In summary, the compounds of present invention block SARS-CoV-2 entry, suggesting that they interfere with early aspects of virus infection. Given that 229E and SARS-CoV-2 entry is mediated by different cellular receptors, it is likely that these compounds interfere with downstream events during virus endocytosis.

Accordingly, it has been demonstrated by present invention that particularly clarithromycin and lexithromycin display a higher antiviral potency against SARS-CoV-2 than other compounds, as, for example, azithromycin in cell culture.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

## BRIEF DESCRIPTION OF THE DRAWINGS

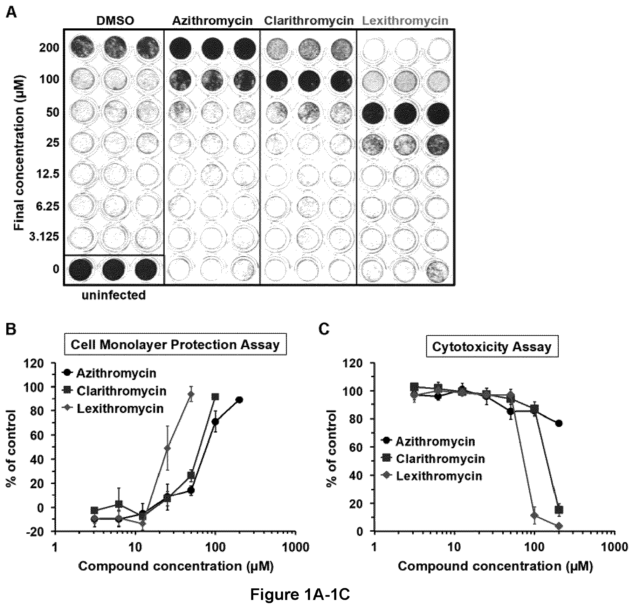

Fig. 1A to 1C. Shows how selected antibiotics protect from the cytopathic effect of SARS-CoV-2 infection. Vero-E6 cells were inoculated at MOI 0.001 with SARS-CoV-2 in the absence or presence of increasing doses of the compounds. (A-B) Seventy-two hours later, cells were fixed, stained with crystal violet and the percentage of remaining biomass was estimated per well. (A) Image of a representative experiment showing protection from SARS-CoV-2 infection. (B) Quantitation of the data shown in A. Data are shown as average and standard deviation of three biological replicates and are expressed as the relative protection in the presence of the compound as compared with the vehicle (DMSO) (C) Toxicity of compounds was determined by quantitation of crystal violet staining of uninfected Vero-E6 cells that were treated in parallel as described in panel A. Data are shown as the average and standard deviation (Mean; SD; n=3).

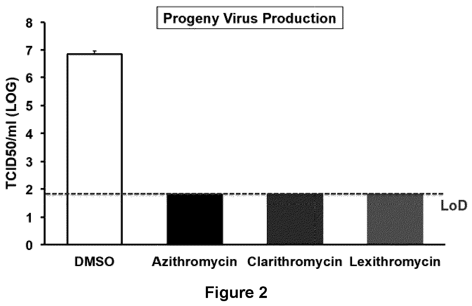

Fig. 2. Shows how selected antibiotics display antiviral activity against SARS-CoV-2 infection. Vero-E6 cells were inoculated at MOI 0.001 with SARS-CoV-2 in the presence of non-toxic concentrations of azithromycin (100 $\mu$M), clarithromycin (100 $\mu$M) or lexithromycin (50 $\mu$M). Forty-eight hours post-infection supernatants were collected and the infectivity titers were determined. Data are expressed as average and standard deviation of the TCID50 values per ml of supernatant obtained in control and compound-treated cells. The lower limit of detection (LoD) of the assay is represented by the discontinued grey line. Note that virus was undetectable in compound-treated conditions, despite the fact that supernatants were diluted below the effective concentrations during the titration assay.

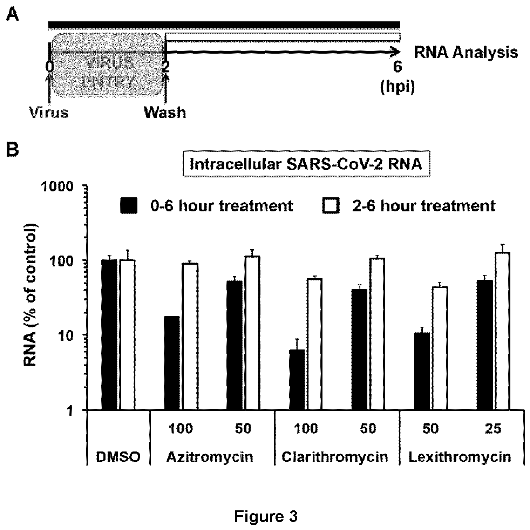

Fig. 3. Shows how selected antibiotics reduce intracellular SARS-CoV-2 RNA accumulation. Vero-E6 cells were inoculated at MOI 5 with SARS-CoV-2 in the presence or absence of the indicated compound concentrations. (A) Diagram explaining the experimental set up used in the experiment. Cells were treated with the compounds either at the time of virus inoculation (black bar) or two hours thereafter (white bar) and compounds were maintained till the end of the experiment. At 6 hours post-infection cell lysates were prepared and the RNA content was analyzed as described in Materials and Methods section. (B) Relative intracellular SARS-CoV-2 RNA quantitation in control and compound-treated samples. Data are expressed as average and standard deviation of biological triplicates. Note that the antiviral effect of compounds is greatly reduced when they are added after virus entry has occurred (white bars) compared to when they are added together with the virus (black bars).

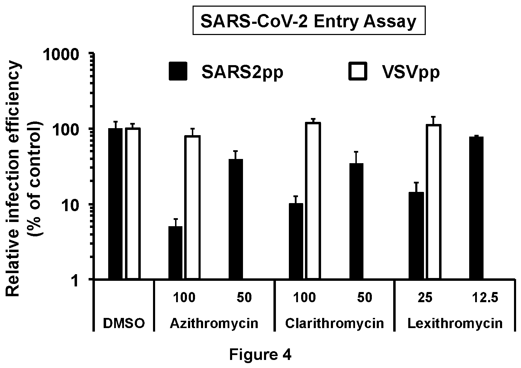

Fig. 4. Shows selected antibiotics inhibit SARS-CoV-2 entry into target cells. Vero-E6 cell monolayers were inoculated with retroviral vectors pseudotyped with SARS-CoV-2 Spike protein (SARS2pp) or VSV envelope glycoprotein (VSVpp) in the absence or presence of increasing doses of the compounds (i.e. 50 and 100 $\mu$M for azithromycin and clarithromycin; and 12.5 and 25 $\mu$M for lexithromycin). Forty-eight hours post-inoculation luciferase activity was determined in whole-cell lysates. Data are expressed as relative luciferase activity values obtained in control and compound-treated cells. Data are shown as average and standard deviation of three biological replicates.

## Examples

### *Materials and methods*

### *In silico* predictions based on Molecular Topology

An *in silico* QSAR strategy based on Molecular Topology (MT) (Zanni R, Galvez-Llompart M, García-Domenech R, Galvez J. 2015. Latest advances in molecular topology applications for drug discovery. Expert Opin Drug Discov 10:945-957) had been previously used to screen two commercial databases: Comprehensive Medical Chemistry (available at: http://www.akosgmbh.de/accelrys/databases/cmc-3d.htm) and Drugbank (available at: https://www.drugbank.ca). In such analysis a discriminant function to differentiate active drugs (protease inhibitors such as lopinavir, amprenavir, indinavir or tipranavir, among others) from inactive ones (non-antiviral drugs: analgesics, antihistamines, hypoglycemiants, etc.) was generated:

$$DF_1 = (0.249 \times SPI) - 5.307 \quad \text{Eq. [1]}$$

N (Total number of compounds) = 111; $\lambda$ (Wilks' lambda) = 0.579; F (Fischer Snedecor) = 79.373; p< 0.00001

SPI: Superpendentic topological index (calculated with alvaDesc software (Mauri A. 2020. alvaDesc: A Tool to Calculate and Analyze Molecular Descriptors and Fingerprints, pp. 801-820. In Ecotoxicological QSARs. Humana, New York, NY, New York, NY)).

Eq. [1] was used to select the potential anti-SARS-CoV-2 drugs screened from the above-mentioned databases, showing values of $DF_1 > 0$.

In table 1 are depicted the results obtained from the virtual screening of selected drugs of the family of antibiotics as potential SARS-CoV-2 protease inhibitors, according to Eq [1], and therefore useful in the treatment of COVID-19.

Table 1

| Drug |
| --- |
| Dalbavancin |
| Nogalamycin |
| Paldimycin |
| Paulomycin |
| Mannosidostreptomycin |
| Bluensomycin |
| Lividomycin |
| Nonactine |
| Diproleandomycin |
| Flurithromycin |
| Clarithromycin |
| Erythromycin |
| Lexithromycin |
| Azithromycin |
| Cethromycin |
| Fomidacillin |
| Neutramycin |
| Cefclidin |
| Cefuroxime |
| Cefbuperazone |
| Mideplanin (mdl 62,873) |
| Teicoplanin |
| Roxithromycin |
| Spiramycin |
| Draxxin |
| Berytromycin |
| Tulathromycin |
| Mirosamicin |
| Oleandomycin |
| Kitasamycin |
| Roquitamycin |
| Maridomycin |

**Potency and cytotoxicity indexes using a 229E-GFP infection assay**

Huh7-Lunet#3 cells (kindly provided by Dr. Thommas Pietschmann; Twincore-Hannover) were maintained subconfluent in complete media [(DMEM supplemented with 10 mM HEPES, 1X non-essential amino acids (Gibco), 100 U/ mL penicillin-streptomycin (Gibco) and 10 % Fetal Bovine Serum (FBS; heat-inactivated at 56 °C for 30 min)].

Huh7-Lunet#3 cells were seeded onto 96-well plates ($1 \times 10^4$ cells/well). The day after, compound stock solutions (10 mM in DMSO) were diluted into complete cell culture media to achieve a final concentration of 100 $\mu$M. The 100 $\mu$M solution was serially diluted 3-fold to achieve decreasing compound concentrations. On the other hand, 229E-GFP virus stock (kindly provided by Dr. Volker Thiel; University of Basel) was diluted in complete media to achieve a final concentration of $3 \times 10^3$ focus forming units (FFU)/ml. One hundred microliters (100 $\mu$l) of the virus dilution were mixed 1:1 with 100 $\mu$l of the compound dilutions to achieve final compound concentrations in a range from 50 $\mu$M to 22 nM and 150 infectious units (FFU) per well in a 96 well plate. One hundred $\mu$l of the mixture was applied onto the Huh7-Lunet #3 cell monolayer in biological replicates and cells were cultured for 72 hours at 33°C in a 5% $CO_2$ incubator. Cells were fixed in a 4% formaldehyde solution in PBS for 10 minutes at room temperature, washed twice with PBS and individual well fluorescence was measured in a SpectraMax iD3 fluorescence plate reader (Molecular Devices). Background subtraction was performed using non-infected wells and signal was normalized to the average fluorescence found in vehicle (DMSO)-treated virus-infected wells. Relative infection efficiency was plotted versus compound concentration to determine the $EC_{50}$ and $EC_{90}$ (effective concentration) values. Once infection efficiency had been determined, plates were stained with a 0.1% crystal violet solution in water-methanol for 30-60 minutes. Then, plates were extensively washed with water and dried before 1% SDS solution in water was added to solubilize crystal violet. Absorbance was measured at 570 nm and background was subtracted from blank wells. Relative well biomass was estimated by calculating the absorbance in each well relative to the average observed in infected cells treated with DMSO. Relative biomass was plotted versus compound concentration to determine the cytotoxic concentration ($CC_{50}$) values.

**SARS-CoV-2 infection assays**

All high and low multiplicity of infection (MOI) experiments were performed by inoculating Vero-E6 cells seeded onto 96-well plates ($2.5 \times 10^4$ cells/well) with the SARS-CoV-2 strain NL/2020 (kindly provided by Dr. R. Molenkamp, Erasmus University Medical Center Rotterdam). Cultures were maintained at 37°C in a 5% $CO_2$ incubator for different lengths of time as indicated in each experiment. Compounds were diluted from 10mM stock solutions in complete media containing 2% FBS to achieve the indicated final concentrations.

*Cell monolayer protection assays:* Vero-E6 cell monolayers were inoculated at MOI 0.001 in the presence of a wide range of compound concentrations (from 200 to 3.125 $\mu$M) in triplicate wells. Seventy-two hours later the cells were fixed and stained using crystal violet, as described above. Stained cells were dissolved in 1% SDS in water and absorbance at 570 nm was used to evaluate the biomass in each well. Uninfected wells and wells infected in the presence of vehicle (DMSO) were used as references for 100% and 0% protection.

*Extracellular progeny virus determination:* Vero-E6 cell monolayers were inoculated at MOI 0.001 in the presence of the indicated compound concentrations. Progeny virus accumulation, present in the supernatants, was determined at 48 hours post-infection by TCID50 determination using the Reed and Muench method (Reed LJ, Muench H. 1938. A simple method of estimating fifty percent endpoints. Am J Trop Med Hyg 27:493-497).

*Intracellular viral RNA quantitation:* Vero-E6 cell monolayers were inoculated at MOI 5 in the presence of the indicated compound concentrations. Six hours later cell lysates were prepared using Trizol reagent (Thermo Scientific). Viral RNA content was determined by RT-qPCR using previously validated sets of primers and probes specific for the detection of the SARS-CoV-2 E gene (Corman VM, et al. 2020. Detection of 2019 novel coronavirus (2019-nCoV) by real-time RT-PCR. Euro Surveill 25:2431) and the cellular $\beta$-actin gene (Gong EY, Smets A, Verheyen N, Clynhens M, Gustin E, Lory P, Kraus G. 2013. A Duplex Real-Time RT-PCR Assay for Profiling Inhibitors of Four Dengue Serotypes, pp. 195-203. In Gong, EY (ed.), Antiviral Methods and Protocols. Humana Press, Totowa, NJ), for normalization purposes. $\Delta$Ct method was used for relative quantitation of the intracellular viral RNA accumulation in compound-treated cells compared to the levels in infected cells treated with DMSO, set as 100%. Time of addition experiments were performed by inoculating Vero-E6 cells with SARS-CoV-2 at MOI 5 in the absence of compounds. Two hour later, virus inoculum was discarded, cells were washed with PBS and they were incubated in the presence of the indicated compound concentrations for four hours. Then viral RNA was extracted and analyzed as described above.

**SARS-CoV-2 Spike protein-pseudotyped retroviral vectors**

Retroviral particle production pseudotyped with different viral envelopes has previously been described (Bartosch B, Dubuisson J, Cosset F-L. 2003. Infectious hepatitis C virus pseudo-particles containing functional E1-E2 envelope protein complexes. J Exp Med 197:633-642; and Mingorance L, Friesland M, Coto-Llerena M, Perez-del-Pulgar S, Boix L, Lopez-Oliva JM, Bruix J, Forns X, Gastaminza P. 2014. Selective inhibition of hepatitis C virus infection by hydroxyzine and benztropine. Antimicrobial Agents and Chemotherapy 58:3451-3460). Packaging plasmids and vesicular stomatitis virus (VSV) G protein expressing plasmid were kindly provided by Dr. F. L. Cosset (INSERM, Lyon). SARS-CoV-2 S expressing plasmid was obtained from Jose Maria Casanovas and Juan Garcia Arriaza (CNB-CSIC). Particles devoid of envelope glycoproteins were produced in parallel as controls.

For SARS-CoV-2 S protein-pseudotyped particle (SARS2pp) entry experiments, Vero-E6 cells ($10^4$ cells/well) were seeded onto 96-well plates the day before. Compounds were diluted in complete media [(DMEM supplemented with 10 mM HEPES, 1X non-essential amino acids (Gibco), 100 U/ mL penicillin-streptomycin (Gibco) and 10 % Fetal Bovine Serum (heat-inactivated at 56 °C for 30 min)] to achieve a 2x concentration. Fifty microliters (50 $\mu$l) of the SARS2pp or VSVpp retrovirus dilutions were mixed 1:1 with 50 $\mu$l of the 2x compound dilutions to achieve the desired final compound concentrations, as indicated in the figure 1. One hundred $\mu$l of the mixture was applied onto the Vero E6 cell monolayer in biological triplicates and cells were cultured at 37°C in a 5% $CO_2$ incubator. Forty-eight hours post-inoculation, cells were lysed for luciferase activity determination using Luciferase Assay System (Promega) and a luminometer. Relative infection values were determined by normalizing the data to the average relative light units detected in the vehicle control cells.

**Statistical analysis**

GraphPad Prism v.5.0a software was used to perform all statistical analysis. All the results were displayed in the graphs as the mean $\pm$ standard deviation. The mean differences between multiple groups were analyzed by One-way ANOVA followed by Dunnett's Multiple Comparison Test. The statistical significance was set as: ns $P > 0.05$; * $P < 0.05$; ** $P < 0.01$; *** $P < 0.001$.

***RESULTS***

**Evaluation of erythromycin and erythromycin-derivatives as candidate drugs against human coronavirus infection.**

As indicated in table 1, a short list of candidate repurposing drugs for the treatment of SARS-CoV-2 from an *in silico* testing of around 15,000 commercially available drugs is reported herein. As a preliminary approach it has been confirmed the antiviral potential of the aforementioned antibiotics against SARS-CoV-2, and has been studied the effective concentration of the compounds capable of blocking infection by a human recombinant model coronavirus bearing a reporter gene (229E-GFP). Infection was not significantly affected by the presence of neither cefuroxime nor erythromycin (Table 2). However, azithromycin, clarithromycin and lexithromycin blocked viral infection in the absence of cytotoxicity at micromolar concentrations (Table 2). In this assay, clarithromycin showed a slightly lower potency than that of the other two active compounds.

Table 2

| Compound | ECso ($\mu$M) | EC$_{90}$ ($\mu$M) | CC$_{50}$ ($\mu$M) |
|---|---|---|---|
| Cefuroxime | > 50 | > 50 | > 50 |
| Erithromycin | > 50 | > 50 | > 50 |
| Azithromycin | 6.1 $\pm$ 2.4 | 18.8 $\pm$ 2.5 | > 50 |
| Clarithromycin | 17.3 $\pm$ 9.2 | 46.3 $\pm$ 4.8 | > 50 |
| Lexithromycin | 3.0 $\pm$ 1.4 | 16.3 $\pm$ 6.0 | > 50 |

**Clarithromycin, azithromycin and lexithromycin interfere with SARS-CoV-2 virus infection.**

Given the antiviral activity in the 229E-GFP infection and in order to evaluate the antiviral potential of the antibiotics in a *bona fide* SARS-CoV-2 infection model, we performed multiple cycle infection experiments (MOI 0.001) in the presence of a range of doses of the vehicle (DMSO) or the compounds that showed antiviral potential against 229E-GFP: azithromycin, clarithromycin and lexithromycin. All three compounds protected target cells from SARS-CoV-2 infection-induced cell death, as shown in figure 1A and 1B, indicating that these compounds are capable of interfering with virus infection sufficiently to protect the target cells from cell death. Using this assay, it is possible to calculate an effective concentration 50 as a surrogate indicator of the relative potency of the compounds. Azithromycin showed the lowest potency as it could only protect completely the cell monolayer at 200 $\mu$M (EC$_{50}$ = 75 $\mu$M). Interestingly, clarithromycin and lexithromycin showed increasing potency and cytotoxicity, protecting around 100% of the cell population at 100 $\mu$M and 50 $\mu$M respectively (EC$_{50}$ = 60 $\mu$M and 25 $\mu$M respectively). Given the relatively high doses required for protection, it has been determined the cytotoxic concentrations of the compounds in Vero-E6 cells and determined that azithromycin was not toxic at any of the tested concentrations and that cytoxicity was proportional to the antiviral activity, being lexithromycin the most toxic compound (Figure 1C).
The protective effect described above was accompanied of a significant reduction in virus progeny production. As shown in figure 2, azithromycin (100 $\mu$M), clarithromycin (100 $\mu$M) and lexithromycin (50 $\mu$M) reduced progeny virus production to undetectable levels, as determined by TCID50 (lower limit of detection = 100 TCID50/ml), in the absence of cytotoxicity (Figure 1C). These results confirm that the protective effect of the compounds is associated with the antiviral activity of the compounds.
Then, single cycle infection experiments were performed in order to determine which of the steps in the virus life cycle is affected by the antibiotics. Treatment with all three compounds significantly reduced viral RNA accumulation in a dose dependent manner, being clarithromycin and lexithromycin more potent than azithromycin (Figure 3, black bars). These results suggest that compounds inhibit early steps in the infection leading to the reduction of intracellular viral RNA. To explore this possibility a time of addition experiment was performed by treating the cells two hours after virus inoculation (white bars). As shown in figure 3 the antiviral effect was markedly reduced when compounds were added after the virus had entered the cells, suggesting that they might interfere with virus cell entry.

**Clarithromycin, azithromycin and lexithromycin inhibit SARS-CoV-2 Spike-mediated viral entry**

To determine the impact of the antibiotics on viral entry, we assessed the ability of retroviral vectors pseudotyped with SARS2 S protein to enter Vero-E6 target cells in the presence of the compounds. Virus infection efficiency suggests that all three antibiotics significantly inhibit S-mediated viral entry (Figure 4). These compounds display the expected relative effectiveness observed in the SARS-CoV-2 infection experiments being lexithromycin the most potent compound (EC$_{50}$ $\cong$ 18 $\mu$M) and clarithromycin and azithromycin displaying, comparable, lower potency (EC$_{50}$ $\cong$ 30 $\mu$M). In parallel, entry of retroviral vectors pseudotyped with VSV G protein (VSVpp) was not inhibited by any of the tested compounds at the highest, non-toxic concentrations, suggesting that the compounds do not interfere with reporter gene expression and that they selectively inhibit S-mediated virus entry.

**Claims**

1. A compound selected from clarithromycin, lexithromycin, cefuroxime, nonactine, cefbuperazone, azithromycin, erythromycin, diproleandomycin. flurithromycin, neutramycin, mirosamicin, fomidacillin, cefclidin, cethromycin, dalbavancin, nogalamycin, paldimycin, paulomycin, mannosidostreptomycin, bluensomycin, lividomycin, mideplanin (Mdl-62,873), teicoplanin, roxithromycin, spiramycin, draxxin, berytromycin, tulathromycin, oleandomycin, kitasamycin, roquitamycin, maridomycin or a pharmaceutically acceptable salt thereof for use in the treatment and/or prevention of a viral infection.

2. The compound for the use according to claim 1, wherein the viral infection is caused by coronaviruses.

3. The compound for the use according to claim 2, wherein the viral infection is caused by SARS-CoV-2.

4. The compound for the use according to claim 3, for the treatment and/or prevention of COVID-19 disease.

5. The compound for the use according to any of claims 1 to 4, wherein the compound is selected from clarithromycin, lexithromycin, cefuroxime, nonactine, cefbuperazone, azithromycin, erythromycin, diproleandomycin. flurithromycin, neutramycin, mirosamicin, fomidacillin, cefclidin and cethromycin.

6. The compound for the use according to any of claims 1 to 5, wherein the compound is selected from clarithromycin, lexithromycin, cefuroxime and erythromycin.

7. The compound for the use according to any of claims 1 to 6, wherein the compound is selected from lexithromycin, clarithromycin and cefuroxime.

8. The compound for the use according to any of claims 1 to 7, wherein the compound is selected from lexithromycin and clarithromycin.

9. A pharmaceutical composition for use in the treatment and/or prevention of a viral infection that comprises a compound according to any of claims 1 to 8 and one or more pharmaceutically acceptable excipients.

10. The pharmaceutical composition for the use according to claim 9, wherein the viral infection is caused by coronavirus.

11. The pharmaceutical composition for the use according to claim 10, wherein the viral infection is caused by SARS-CoV-2.

12. The pharmaceutical composition for the use according to claim 11, for the treatment and/or prevention of COVID-19 disease.

13. An antiviral that comprises a compound according to any of claims 1 to 8 or a pharmaceutical composition according to any of claims 9 to 12.

Figure 1A-1C

Figure 2

**A**

**B**

Figure 3

Figure 4

**European Patent Office**

Europäisches Patentamt
European Patent Office
Office européen des brevets

# EUROPEAN SEARCH REPORT

Application Number

EP 20 38 2570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CN 111 297 838 A (NINGBO HEKANG BIOMEDICAL TECH CO LTD) 19 June 2020 (2020-06-19) * the whole document * | 1-13 | INV. A61K31/546 A61K31/7048 A61P11/00 A61P31/14 |
| X | US 2018/318328 A1 (FATHI REZA [US] ET AL) 8 November 2018 (2018-11-08) | 1,5-9,13 | |
| Y | * paragraphs [0079], [0171] - [0184], [0202] * | 1-13 | |
| X | WO 2017/151120 A1 (EMERGING VIRAL THERAPEUTICS LTD [CN]; LAU JOHNSON [US]) 8 September 2017 (2017-09-08) | 1,5-9,13 | |
| Y | * paragraphs [0005], [0028] - [0029]; claims 1-3 * | 1-13 | |
| X | US 2006/062762 A1 (WOODWARD JOHN R [US]) 23 March 2006 (2006-03-23) | 1,5-9,13 | |
| Y | * paragraph [0036]; claim 1 * | 1-13 | |
| Y | AU 2020 100 641 A4 (BALASUBRAMANIAM VAIDYANATHAN DR [AU] ET AL.) 4 June 2020 (2020-06-04) * the whole document * | 1-5,9-13 | |
| X | CN 107 281 210 A (INST MATERIA MEDICA CAMS) 24 October 2017 (2017-10-24) | 1-3,5,9, 10,13 | TECHNICAL FIELDS SEARCHED (IPC) A61K A61P |
| Y | * the whole document * | 1-3,5,9, 10,13 | |
| X | CN 105 456 283 B (SUN YAT-SEN UNIV; MEIZHOU PEOPLE'S HOSPITAL) 28 August 2018 (2018-08-28) | 1,5-9,13 | |
| Y | * claim 1 * | 1-13 | |

The present search report has been drawn up for all claims

| Place of search | Date of completion of the search | Examiner |
|---|---|---|
| Munich | 16 November 2020 | Estañol, Inma |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 20 38 2570

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2020

| Patent document cited in search report | | Publication date | Patent family member(s) | | Publication date |
|---|---|---|---|---|---|
| CN 111297838 | A | 19-06-2020 | NONE | | |
| US 2018318328 | A1 | 08-11-2018 | AU | 2015334590 A1 | 18-05-2017 |
| | | | AU | 2019271958 A1 | 19-12-2019 |
| | | | BR | 112017008220 A2 | 09-01-2018 |
| | | | CA | 2965258 A1 | 28-04-2016 |
| | | | CN | 107106525 A | 29-08-2017 |
| | | | EP | 3209290 A1 | 30-08-2017 |
| | | | JP | 6625631 B2 | 25-12-2019 |
| | | | JP | 2017531673 A | 26-10-2017 |
| | | | KR | 20170070234 A | 21-06-2017 |
| | | | SG | 11201703307W A | 30-05-2017 |
| | | | US | 2016113954 A1 | 28-04-2016 |
| | | | US | 2018318328 A1 | 08-11-2018 |
| | | | US | 2020155585 A1 | 21-05-2020 |
| | | | WO | 2016063134 A1 | 28-04-2016 |
| WO 2017151120 | A1 | 08-09-2017 | AU | 2016396042 A1 | 20-09-2018 |
| | | | CA | 3016119 A1 | 08-09-2017 |
| | | | CN | 109069471 A | 21-12-2018 |
| | | | EP | 3423051 A1 | 09-01-2019 |
| | | | RU | 2018131136 A | 01-04-2020 |
| | | | TW | 201731496 A | 16-09-2017 |
| | | | US | 2019054060 A1 | 21-02-2019 |
| | | | WO | 2017151120 A1 | 08-09-2017 |
| US 2006062762 | A1 | 23-03-2006 | NONE | | |
| AU 2020100641 | A4 | 04-06-2020 | NONE | | |
| CN 107281210 | A | 24-10-2017 | NONE | | |
| CN 105456283 | B | 28-08-2018 | NONE | | |

EPO FORM P0459

For more details about this annex : see Official Journal of the European Patent Office, No. 12/82

## REFERENCES CITED IN THE DESCRIPTION

### Non-patent literature cited in the description

- **WANG J.** Fast Identification of Possible Drug Treatment of Coronavirus Disease -19 (COVID-19) Through Computational Drug Repurposing Study. *J Chem Inf Model,* 2020, https://doi.org/10.1021/acs.jcim.0c00179
- **GAUTRET P ; LAGIER JC ; PAROLA P ; HOANG VT ; MEDDEB L et al.** Hydroxychloroquine and azithromycin as a treatment of COVID-19: results of an open-label non-randomized clinical trial. *Int J Antimicrob Agents, https://doi.org/10.1016/j.ijantimicag.2020.105949*
- **ZANNI R ; GALVEZ-LLOMPART M ; GARCIA-DOMENECH R ; GALVEZ J.** Latest advances in molecular topology applications for drug discovery. *Expert Opin Drug Discov,* 2015, vol. 10 (9), 945-957
- **WANG J ; LAND D ; ONO K ; GALVEZ J ; ZHAO W et al.** Molecular topology as novel strategy for discovery of drugs with aβ lowering and anti-aggregation dual activities for Alzheimer's disease. *PloS one,* 2014, vol. 9 (3
- **MCGONAGLE, D. ; O'DONELL, J. et al.** Immune mechanisms of pulmonary intravascular coagulopathy in COVID-19 pneumonia. *Lancet Rheumatol,* 2020, https://doi.org/10.1016/S2665-9913(20)30121-1
- **TAY, M.Z. ; POH, C.M. ; RÉNIA, L. et al.** The trinity of COVID-19: immunity, inflammation and intervention. *Nat Rev Immunol,* 2020, vol. 20, 363-374
- **ZANNI R ; GALVEZ-LLOMPART M ; GARCÍA-DOMENECH R ; GALVEZ J.** Latest advances in molecular topology applications for drug discovery. *Expert Opin Drug Discov,* 2015, vol. 10, 945-957
- alvaDesc: A Tool to Calculate and Analyze Molecular Descriptors and Fingerprints. **MAURI A.** Ecotoxicological QSARs. Humana, 2020, 801-820
- **REED LJ ; MUENCH H.** A simple method of estimating fifty percent endpoints. *Am J Trop Med Hyg,* 1938, vol. 27, 493-497
- **CORMAN VM et al.** Detection of 2019 novel coronavirus (2019-nCoV) by real-time RT-PCR. *Euro Surveill,* 2020, vol. 25, 2431
- A Duplex Real-Time RT-PCR Assay for Profiling Inhibitors of Four Dengue Serotypes. **GONG EY ; SMETS A ; VERHEYEN N ; CLYNHENS M ; GUSTIN E ; LORY P ; KRAUS G.** Antiviral Methods and Protocols. Humana Press, 2013, 195-203
- **BARTOSCH B ; DUBUISSON J ; COSSET F-L.** Infectious hepatitis C virus pseudo-particles containing functional E1-E2 envelope protein complexes. *J Exp Med,* 2003, vol. 197, 633-642
- **MINGORANCE L ; FRIESLAND M ; COTO-LLERENA M ; PEREZ-DEL-PULGAR S ; BOIX L ; LOPEZ-OLIVA JM ; BRUIX J ; FORNS X ; GASTAMINZA P.** Selective inhibition of hepatitis C virus infection by hydroxyzine and benztropine. *Antimicrobial Agents and Chemotherapy,* 2014, vol. 58, 3451-3460